# EUROPEAN PATENT APPLICATION

(11) **EP 1 733 746 A2**
(43) Date of publication of application: **20.12.2006**
(21) Application number: 06397012.3
(22) Date of filing: 13.06.2006
(51) Int. Cl.: A61L 27/56, A61L 27/58, A61L 27/44

(54) **Bioabsorbable implant having a varying characteristic**

(30) Priority: 13.06.2005 FI 20055304
(71) Applicant: BIORETEC OY, 33720 Tampere (FI)
(72) Inventor: Ashammakhi, Nureddin, 33100 Tampere (FI); Törmälä, Pertti, 33300 Tampere (FI); Hupa, Mikko, 20720 Turku (FI); Ylänen, Heimo, 20810 Turku (FI)
(74) Representative: Hakola, Unto Tapani

(57) **Abstract**

A bioabsorbable implant or its part consists of a structure having a varying characteristic in at least in one direction defining a gradient corresponding this characteristic. The structure comprises bioabsorbable polymer and another substance within or next to said bioabsorbable polymer. The structure is constituted of a wound or folded blank (1) having a direction of variation (L) of a characteristic (Ch) which is at least one of the following: concentration of the other substance, species of the other substance, type of the bioabsorbable polymer, or porosity (size of openings in the blank), said structure having a winding axis or fold lines substantially perpendicular to said direction of variation (L). This results in said varying characteristic in the structure in said at least one direction defining the gradient and traversing superposed layers formed of sections of the blank (1) that are successive in said direction of variation (L).

## Description

### FIELD OF THE INVENTION

The invention relates to a bioabsorbable implant having a varying characteristic at least in one direction. Especially the invention relates to an implant having porous structure, preferably interconnected porosity. Moreover, the implant is preferably a load-bearing medical device structure comprising bioabsorbable polymer and intended for hard tissue (osteochondral) applications.

### BACKGROUND OF THE INVENTION

Various materials have been developed to act as scaffolds for bone tissue generation or regeneration. They have been called so because they offer a medium to which cells can attach after implantation in a body. These scaffolds contain bioabsorbable polymers, such as polyglycolide, polyglycolide/polylactide and polylactide, to name a few.

An example of a biocompatible implant is shown in US patent 5,084,051. The implant is made of biocomposite material comprising at least one bioceramic component layer and at least one material component layer, which has been manufactured of at least one polymer, both components having certain porosity.

US patent 6,579,533 describes a bioabsorbable drug delivery material comprising synthetic bioabsorbable polymeric matrix, antibiotic and bioactive glass dispersed in the polymeric matrix. The document mentions a possibility to spin drug releasing materials to fibers which can be formed to knitted or woven fabrics for example.

US patent 5,626,861 is a good example of a conventional technique for obtaining 3-dimensional macroporous polymer matrices for use as bone graft or implant material. Mixing a polymer solution, hydroxyapatite particles and inert particles, which are removed by leaching after the solvent of the polymer has evaporated, forms the composite. The technique requires many processing steps and use of organic solvents. The inner porosity cannot be thoroughly controlled through this procedure.

Publication WO 02/08320 shows a simpler technique avoiding the use of solvents, but it still requires the use of the special "porogen" substance, which must be removed from the composition to create the porosity.

An example of a biocompatible implant for surgical implantation is shown in US published patent application no. 2002/0143403 and corresponding publication WO 02/053105. This implant comprises a matrix of a resorbable thermoplastic-ceramic composition, the matrix having a pore size and porosity effective for enhancing bone growth adjacent the composition. The implant structure is made by using ribbon or filament deposition process. The ribbons or filaments of extruded composition are deposited layer upon layer onto the work or support surface in a predetermined pattern to form an object of desired size and shape and having the desired porosity characteristics, using a special extrusion freeform (EFF) process. One material for the composition is a blend of thermoplastic polymer and calcium phosphate.

US patent 5,711,960 shows another three-dimensional structure consisting of a three-dimensional fabric made by a special weaving or knitting technique.

US patent 6,534,084 describes a three-dimensional interconnected open cell porous foam. The purpose is to provide a scaffold in the form of a biocompatible gradient foam that has a substantially continuous transition in at least one characteristic, for example pore architecture, as an alternative to porous structures having isotropic or random microstructure. The structure is made by lyophilization of a solvent-polymer-mixture under controlled conditions.

US patent 5,153,002 describes a biocompatible and/or biodegradable implant for the substantially constant release, by diffusion, of a therapeutic agent. The implant is a cylindrical body where the diffusion takes place only through the end wall, and the therapeutic agent has different concentration along the axis of the cylinder. This is done by sedimentation of the agent by centrifugal force when the matrix is still in a liquid state.

US patent 4,351,069 describes a coated prosthetic device having a porosity or density gradient in the sintered palstci coating only. The higher porosity at the outer surface facilitates bone ingrowth while the lesser porosity and higher density of the inner surface provides better adhesion to the actual load bearing component. Publication WO 2004/026361 discloses implantable medical devices having a polymer gradient coating releasing at least one pharmaceutical compound. The gradient coating is prepared by sequential layering of different compositions.

US patent 6,454,811 deals with application of solid free-from fabrication methods, such as so-called three dimensional printing process, to the creation of composite devices for tissue engineering. The technique makes it possible to create a gradient in one or more of the following: materials, macroarchitecture, microarchitecture, or mechanical properties.

International publication WO 2004/049904 discusses the formation of porous scaffolds for tissue engineering in repair of bone and cartilage defects from bioactive glass fibers and resorbable polymer fibers. The scaffold can have a gradient in porosity, which can be achieved for example through the weaving and subsequent three dimensional assembly of the weaves which creates a three-dimensional structure with layers of weaves in which the subsequent layers have different weaving characteristics.

Patent US 6,113,640 and corresponding patent EP 988001 in turn show a reconstructive bioabsorbable joint prosthesis comprising a cylindrical fibrous porous joint spacer that is formed from a strip of fabric by wrapping. The fabric is made of fibers of a biodegradable polymer, co-polymer, polymer mixture or composition.

The fabrication methods of prior art require either a sophisticated expensive manufacturing apparatus for 3-dimensional forming to provide an accurate gradient in the interior of an implant or, if simpler, do not allow the creation of the gradient in a controlled manner or require prefabricated layers made separately before the formation of the implant.

### SUMMARY OF THE INVENTION

The purpose of the invention is to provide a gradient implant having at least one characteristic that varies along at least one axis with a great accuracy and that can be manufactured with simpler methods than before. The implant comprises bioabsorbable polymer and another substance within or next to said bioabsorbable polymer. Said other substance does not belong to bioabsorbable polymers and is of any type discussed hereinbelow. The characteristic that varies along said at least one axis is the concentration or species of said other substance or the type of the bioabsorbable polymer. The concentration of the other substance can be defined as proportion of the other substance to the bioabsorbable polymer (w/w). The implant of the invention is especially porous, in which case the said varying characteristic can also be porosity.

The implant is preferably porous, mechanically reliable, biocompatible and bioabsorbable, and intended to achieve successful tissue, for example hard tissue (cartilage tissue and/or bone) generation, regeneration, repair, fixation, separation, replacement, reconstruction or augmentation. The composite structure is composed in a special multilayered fashion of a plurality of individual layers where the said characteristic varies layer by layer so that the gradient representing said variation exists in a direction substantially perpendicular to the planes of said layers. The individual layers consist of the same continuous blank wound in spiral form of folded in some other form so that the successive sections of the blank form the individual layers next to each other. The blank is manufactured so that the said at least variable characteristic varies along the length of the blank (in the direction of succession of the separate successive sections). The characteristic in question can be the above mentioned concentration or species of the other substance or the type of the bioabsorbable polymer in said blank.. If porosity gradient is desired, the blank must have openings, and the characteristic is then the size of openings in the blank.

The invention makes it possible also to include elongate continuous or non-continuous reinforcing elements (filaments or staple fibers) in the structure of the implant. These elements may be bioactive ceramic elements, for example bioactive glass. Said reinforcing elements constitute said other substance that exists in the implant next to or within the bioabsorbable polymer and that can have the variation corresponding to the gradient.

Many other active agents can be incorporated in the implant to constitute the other substance. For example, the agent can be a bioactive agent in a bioabsorbable polymer matrix, and the characteristic providing the desired gradient can be the concentration or kind of the bioactive agent within the polymer matrix. The other substance can also be an additive, such as a marker, color, indicator or tracer.

If porosity is the characteristic, the blank can be a sheet formed of elongate elements where openings are formed between the elements, e.g. a textile product. In the final implant the openings create interconnecting porosity inside the implant connected to the outer surface of the implant. The size of openings can vary along the length of the blank when the blank is straight and uniformly tensioned. When the openings become deposited next to each other in the successive layers, a desired and accurately determined gradient in the pore size and interconnecting porosity is obtained at the same time.

The structure provided by the textile layers comprises thus interconnecting channels and pores which provide a suitable environment and scaffold for *in vivo* integration into body tissues upon implantation, but also for incorporating active agent and/or cells at *in vitro* stage prior to implantation, for example such agents that can not or that should not be embedded in the matrix of the bioabsorbable polymer.

The structure of the implant containing at least one gradient is preferably load-bearing as such, i.e. it is not merely a coating arranged over a load-bearing body.

The implant according to the invention is especially intended for *in situ* tissue engineering. It is intended for tissue generation, tissue repair, tissue support, tissue fixation or tissue augmentation, filling, replacement, reconstruction or controlled repair, controlled healing, or controlled regeneration. The implant of the current invention is preferably of the type having reliable mechanical strength to be effective for use in the management of hard tissues such as bone, cartilage and their composites in the above-mentioned applications in mammals, especially in human body (osteochondral surgery).

If the varying characteristic or property resulting therefrom can be expressed by numerical values, the gradient can be one of the following in terms of the value in the direction of the gradient: gradually increasing, gradually decreasing, gradually increasing and then decreasing (showing a maximum), and gradually decreasing and then increasing (showing a minimum). This means that the change along said axis is not random but follows a predetermined pattern. The characteristics that can be expressed in numerical values are concentration of the other substance (its ratio to bioabsorbable polymer w/w), porosity (pore size), and bioabsorption rate resulting from the type of the bioabsorbable polymer.

The implant can contain more than one characteristic that provides the gradient, i.e. there can be two or more different gradients. For example one of the gradients can be related to porosity and the other to concentration or species of the other substance or the type of the bioabsorbable polymer.

The implant, once placed in a living body, can have graded functionality, depending on the type of the gradient.

The implant may also be substantially non-porous and formed analogically from a sheet-like blank where at least one characteristic selected from the concentration of the other substance, the species of the other substance and the type of the bioabsorbable polymer varies along the blank. The other substance can exist within the sheet made of bioabsorbable polymer.

### BRIEF DESCRIPTION OF THE DRAWINGS

- Fig. 1: shows the basic principle of the implant of the invention in schematical representation,
- Fig. 2: shows the structure of a blank in one embodiment in top plan view,
- Fig. 3: shows the structure of an implant made from the blank of Fig. 2 in cross-section,
- Fig. 4: shows the structure of a blank in another embodiment in top plan view, and
- Fig. 5: shows the structure of an implant made from the blank of Fig. 4 in cross-section.

### DETAILED DESCRIPTION OF THE INVENTION

### Terminoloay

The term "fiber" used in this specification is meant to describe continuous filament and staple fiber (discontinuous fiber of limited length, typically under 10 mm).

The term "fibrous element", used for the basic constituent of the blank in some embodiments, refers to an elongate flexible element that can be mechanically made into a two-dimensional textile structure but not necessarily constituted of individual fibers.

The term "ceramic" refers to any material of non-metal and inorganic origin (definition agreed on at concensus conferences on biomaterials).

The term "biomaterial" refers to material intended to have an interface with biological systems and that is used to evaluate, treat, augment or replace any tissue, organ or function of the body (definition agreed on at concensus conferences on biomaterials).

The term "bioactive" refers to bioactive materials designed to elicit or modulate biological activity (definition agreed on at concensus conferences on biomaterials).

### General structures

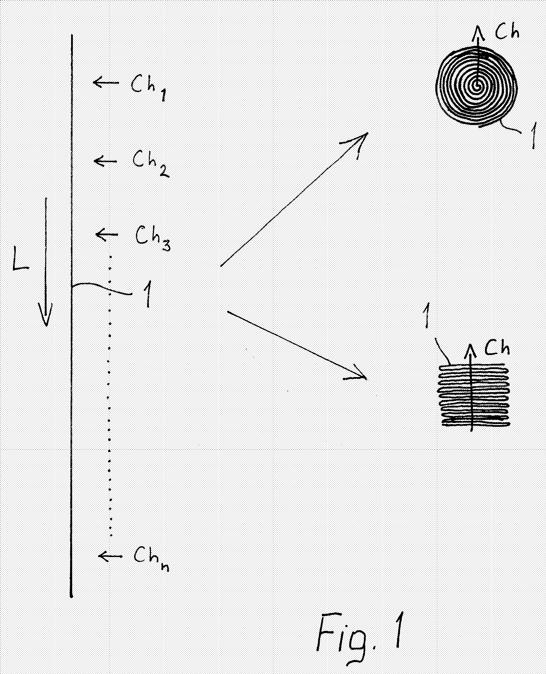

Fig. 1 shows the basic principle of the invention. The starting material of the implant is a flexible blank 1, preferably of constant width and thickness. The blank comprises at least bioabsorbable and biocompatible polymer (either one type of such polymer or a blend of such polymers). There is one characteristic, designated Ch, that varies along the length of the blank running perpendicularly to the width. The difference in the characteristic is denoted with different indexes 1...n along the length, and the direction of variation is denoted with L.

When the blank is wound into cylindrical form (right-hand side of the figure, upper part) so that its successive sections along the length form superposed windings in the cylindrical body, the characteristic Ch will vary in the radial direction of the cylindrical body, i.e. form the core (cylinder axis) towards the surface. By constructing the blank so that the characteristic has a desired variation along its length, the final implant shape will have accurate gradient form its center to the surface and corresponding graded functionality in the same direction.

The result of the winding need not be a cylindrical body. The winding can have another shape, for example oval, rectangular with more or less flat shape, or even square, with more or less rounded corners or edges. Common to all these shapes are superposed layers constituting of successive sections of the blank, and the gradient will rn from the enter to the surface in these shapes as well. The shape can be also curved, i.e. the laps can follow a crescent shape. Substantially cylindrical body is preferred if rod-shaped implants are to be manufactured.

The body can also be hollow, which is achieved by winding the blank 1 around a mandrel or core which leaves an empty space inside the body after removing the wound structure from around the mandrel or core. Tubular implants having an inner surface and outer surface can thus be formed. The gradient runs form the inner surface to the outer surface in the implants of this type.

In the lower part of the right-hand side, another alternative is shown where the blank is folded so that the successive sections become deposited one top of the other when they are directed alternately to both sides, creating alternately folds on both sides. In the resulting structure the gradient will run from one surface to the opposite surface. The resulting structure may also be curved, i.e. the layers may follow an arcuate path.

The shapes shown in Fig. 1 can be consolidated to a structure where the layers are fixed to each other by using the properties of the bioabsorbable polymer. The bioabsorbable polymer is preferably a thermoplastic polymer. Said bioabsorbable thermoplastic polymer matrix can be used to bind together the layers formed of said successive sections of the blank by application of heat and possibly pressure and subsequent cooling. In this way a stiff, load bearing structure can be obtained form the wound or folded prefabricate. Depending on the initial composition of the blank, the thermoplastic bioabsorbable material can be melted into a continuous matrix phase in the direction of gradient.

The blank and the resulting implant comprises additionally another substance that exists next to or within the bioabsorbable polymer. The structure comprising the bioabsorbable polymer and the other substance can be termed "composite" or "mixture".

The preceding description presented the general idea of the invention. It is understood that the varying characteristic Ch can be selected from at least one of the following: concentration of the other substance, species of the other substance, type of the boabsorbable polymer, or porosity. In the following, some more detailed examples are given.

### Porous implant structure

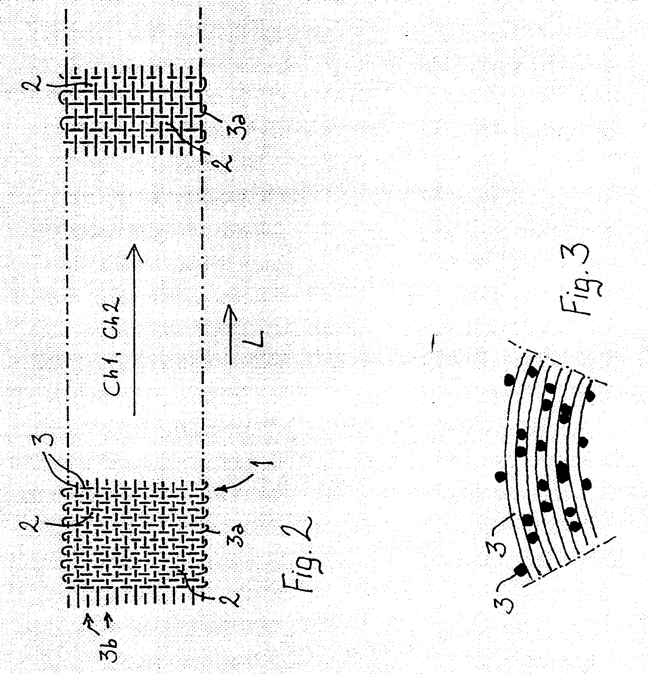

Fig. 2 shows a blank 1 by which an implant of controlled interconnected porosity can be formed. The blank 1 is in the form of a foraminous structure, where the size and/or distribution of the openings 2 is the characteristic Ch that varies along its length (arrow L). The openings of different sizes and/or distribution will thus be laid on top of each other in successive layers, forming a porosity gradient in the direction perpendicular to the layers (in case of a cylindrical body, in radial direction). A part of the final implant is shown in Fig. 3 in cross-section.

The openings 2 can in principle be formed in any possible way in the blank 1, and the resulting implant will have the graded porosity according to the variation of the openings along the length of the blank.
Fig. 2 shows an embodiment where the blank is made of continuous fibrous elements 3, and said openings 2 are defined by portions of said continuous fibrous elements 3 extending substantially in the direction of the plane of the blank 1. When the different sections of said blank end up on top of each other, said openings 2 in different sections form the porous structure. The blank can be a mesh, woven fabric, braid or knitted frabric or any other textile structure where the continous fibrous elements 3 define openings 2 therebetween.

The blank can contain one set of fibrous elements running in one direction and another set of fibrous elements of different composition running crosswise to them. If said one set of fibrous elements run in a direction perpendicular to the length of the blank, the alteration of these elements along the length of the blank (direction L) may be the characteristic Ch. For example if the spacing of the elements varies, this will result in porosity gradient as will be shown hereinafter. Simultaneously there may be another characteristic which changes in the direction L independently of the alteration of the set of fibrous elements running perpendicularly to the direction L. This may be achieved by different types of fibrous elements in this set of elements. Along this direction L, there may be a transition of fibrous elements of one type to the fibrous elements of another type, or transition of the proportion of two different types of elements.

The changing characteristic may, additionally to or instead of the above-mentioned different types of fibrous elements, be achieved by some characteristic of the other set of fibrous elements extending in the direction L, for example by composition of the fibrous elements that changes along their length and consequently along the length of the blank 1.

Fig. 2 shows a structure of a woven fabric, where the openings 2 are between the weft yarns 3a (said one set of fibrous elements running along the length) and warp yarns 3b (the other set of fibrous elements). The warp yarns 3b are made of or contain bioabsorbable polymer and the weft yarns 3a are made of or contain bioactive ceramic. The mutual spacing of the weft yarns 3a varies along the length of the blank (arrow L), which creates varying size and distribution of the openings 2 along the same direction (first characteristic Ch1). Because the varying spacing of the weft yarns 3a affects also distribution of the bioactive ceramic along the length of the blank (if the same weft yarn 3a is used throughout the blank 1 as is usual in woven structures), the concentration of the bioactive ceramic also varies in the same direction and constitutes another characteristic Ch2 that makes up another gradient in the final implant. If the content of the bioabsorbable polymer in the warp yarns 3b is constant along the length L, the ratio of the bioactive ceramic to the bioabsorbable polymer and consequently its concentration will vary. These two characteristics (openings and concentration) are in this case not independent of each other but interdependent. Another alternative is to alter the composition of the weft yarn 3a (corresponding to the concept of different types of fibrous elements in the set of elements running perpendicularly to direction L), either by using yarns of different compositions as weft yarns 3a successively along direction L, or by using one single weft yarn where the composition varies as function of its length. In this case the first characteristic Ch1 (size of openings 2) and the other characteristic Ch2 (composition, such as contents or kind of bioactive glass) can be arranged to change independently of each other as function of length (direction L) of the blank 1.

The warp yarns 3b can have differing composition along their lengths which coincide with the length of the blank 1 (direction L) and consequently result in a composition gradient in the implant. This differing composition, which may be for example concentration of a bioactive agent or material in the yarn, can constitute a third characteristic Ch3. The bioactive agent or material can be for example dispersed or dissolved in the bioabsorbable polymer matrix material of the warp yarn 3b. The bioactive agent or material can be for example a cytokine or pharmaceutical agent (drug), that will be released in the body when the bioabsorbable polymer degrades and/or a bioactive ceramic of another kind than the bioactive ceramic in the weft yarn 3a. Antmicrobial agents and anti-inflammatory agents are non-limiting examples of the above-mentioned drugs. Additionally to or instead of the above-mentioned characteristic, the kind of bioabsorbable themoplastic polymer in the warp yarns 3b may be the said characteristic, whereby the polymers may differ in rate of bioabsorption. In the practice, this type of yarn can be made by using different bioabsorbable polymer staple fibers along the length of the yarn, or different polymer in successive sections of a continuous phase of the yarn (in filament(s) or coating of the yarn).

In Fig. 2, as one practical example only, a blank 1 is shown where the spacing of weft yarns 3a is smaller in the first end of the blank (forming the core of the implant) and larger in the opposite end (forming the outer surface), this resulting in graded functionality of porosity where the pore size increases from the core to the exterior surface.

The gradients related to bioabsorbale thermoplastic polymer and bioactive glass can be arranged in the blank 1 such that the faster-degrading bioabsorbable polymer and slower-degrading bioactive glass is deeper in the implant (for example closer to the core) and the faster-degrading bioactive glass and slower-degrading bioabsorbable polymer is on the outer surface of the implant, these characteristics changing gradually from inside the implant to the surface.

When the different sections having the two-dimensional structure as shown in Fig. 2 become laid on top of each other, they can be joined permanently to each other by the bioabsorbable polymer of the warp yarns 3b. When the prefabricate consisting of wound or folded blank is subjected to heat, the thermoplastic bioabsorbable polymer melts and on cooling binds the layers (different successive blank sections) together.

Thanks to the gaps remaining between the different fabric layers laid on top of each other, which is due to the wave-like course of at least some yarns along the plane of the blank and resulting surface irregularities of the blank, the structure has some porosity in the direction of the interface between the layers.

Variations to the structures of the Figs. 2 and 3 are possible. For example the textile structure can be formed in a different way than by weaving, for example by knitting. In this case the openings 2 are formed by the loops of the yarn forming the knit. The knitted fabric can have varying loop sizes along the length of the blank 1. The yarn has bioabsorbable thermoplastic polymer at least as one component so that it can be used for joining the different sections of the blank 1. The yarn contains preferably both bioabsorbable thermoplastic polymer and bioactive ceramic, especially bioactive glass, and all yarns discussed hereinbelow that combine these two components can be used. In the structure there can also be porosity in the direction of the interface between the layers because of the irregularities of the surface of the knitted fabric. The composition of the yarn used for the knitted layer can also be vary in its longitudinal direction, this resulting in gradual variation of the respective characteristic Ch in the longitudinal direction of the blank (if the courses run crosswide to the length of the blank), and finally in the gradient of that characteristic Ch. The composition characteristic may be for example the content and/or kind of bioactive ceramic, especially bioactive glass. It is possible that there is variation both in the macrostructure (loop size) and concentration of a component (composition of the yarn) which are independent of each other, i.e. the variation of one characteristic can be performed independently of the variation of the other.

### Structural varieties of continuous fibrous elements

In the foregoing example one set of fibrous elements is made of bioactive ceramic, especially bioactive glass, and the other set of fibrous elements is made of bioabsorbable thermoplastic polymer.

It is possible that in said one set of fibrous elements (weft yarns 3a in the example of Fig. 2), the fibrous bioactive ceramic exists in filament or staple fiber form. If the filaments are used, the element can be formed from one filament or by combining a plurality of filaments. If staple fibers are used, the fibrous element can be made by spinning from the fibers. It is also possible that said one set of fibrous elements contains another component additionally to the bioactive ceramic, for example bioabsorbable thermoplastic polymer that contributes to the fixation of the structure by heat. If another component is to be included in the fibrous elements, they may be brought in filament or staple fiber form and incorporated in the fibrous element by combining with bioceramic filaments or spinning with bioceramic staple fibers, respectively. The component may be bioabsorbable thermoplastic polymer in staple fiber or filament form. One or several bioceramic filaments may also be covered or impregnated with bioabsorbable thermoplastic polymer using for example cable covering technique. It is possible to make the type of the bioabsorbable polymer vary along the length of the weft yarn, in which case the the characteristic that varies along the length L will be related to the type of polymer, for example different chemical compositions (which may result in different bioabsorption rates), or chemically same polymers with differing biaobsorption rates.

Finally, if a bioactive ceramic is to be included in said one set of fibrous elements (weft yarns 3a in the example of Fig. 2) in other than fibrous form (filament(s) or staple fibers), it can be dispersed therein as particles. In this case the matrix where the ceramic material is dispersed is of a bioabsorbable thermoplastic polymer in filament form. If the ceramic to be dispersed in form of particles in the matrix material is bioactive glass, bioactive glass without fiber-forming properties can be used.

In said other set of fibrous elements (warp yarns 3b in the example of Fig. 2) the bioabsorbable thermoplastic polymer exists in filament or staple fiber form. If the filaments are used, the element can be formed from one filament or by combining a plurality of filaments. If staple fibers are used, the fibrous element can be made by spinning from the fibers. If variation in the bioabsorbable polymer component is desired along the length of the yarn, different polymer staple fibers are used during the spinning procedure in time-discrete fashion. If another component is to be included in the fibrous elements, they may be brought in filament or staple fiber form and incorporated in the fibrous element by combining with bioabsorbable thermoplastic polymer filaments or spinning with bioabsorbable thermoplastic polymer staple fibers, respectively. This additional component of the other set of fibrous elements may be bioactive ceramic of another type than the bioactive ceramic in said one set of fibrous elements. The bioactive ceramic may in this case act as reinforcement of the other set of fibrous elements. If this reinforcement is a continuous filament, the bioabsorbable thermoplastic polymer component may in this case be a coating covering one or plurarity of such filaments. The fibrous elements may in this case be made by covering or impregnating with bioabsorbable thermoplastic polymer using for example cable covering technique. If variation in the bioabsorbable polymer component is desired in the longitudinal direction of the yarn, different polymer raw materials are used during the covering or impregnation procedure in time-discrete fashion.

It may be of advantage that part of the bioactive ceramic material, such as bioactive glass, is present on the surface of the fibrous element, for example on the surface of said one set of fibrous elements running crosswise to the length of the blank (for example weft yarns 3a of Fig. 2). In the final structure of the device it will mean that the ceramic will be exposed in the pores, which enhances the interaction of the bioactive ceramic component with its environment, through the pores also with the outside of the implant. One advantageous structure of the yarn for this purpose is a yarn containing bioactive ceramic staple fibers whose ends, owing to the stiffness of the fibers, spread sideways from the main running direction of the yarn in a "hairy" fashion, thus penetrating into the pores and channels inside the structure.

Any of the above-mentioned flexible fibrous elements constituted of one material (bioactive ceramic such as bioactive glass, or bioabsorbable thermoplastic polymer) or being a combination of materials (hybrid yarns) can be knitted, woven, braided or processed by another textile manufacturing method into a blank with a wide surface area. Any hybrid yarn described above can be used especially as the yarn of the knitted fabric if it is desired that the implant made of a knitted blank contains both thermoplastic bioabsorbale polymer and bioactive ceramic.

The substance other than the bioabsorbable polymer that is made to vary with respect of its concentration or type in the porous structure discussed hereinabobe can be a cytokine and/or pharmaceutical agent (drug), a bioactive ceramic, a peptide, or a polynucleotide. It need not necessarily have biological effect but it can be an additive helpful in other respects such as evaluating the functioning of the implant. It can be a marker, color, indicator, tracer or other additive.

In case of bone repair and healing (fixation, regeneration/generation, augmentation) clinical end applications, an example of one important bioactive agent is anti-osteolytic agent that inhibits bone resorption, such as agents that interfere with inflammation or agents that inhibit osteoclasts (anti-osteoclastic), can be included in the matrix material of the implant of the current invention. This can be done in the graded fashion as explained above so that the concentration of the agent has a desired profile along the gradient. An important example of this group of agents belongs to a group called bisphosphonates (see also Watts WB:Bisphosphonates therapy for postmenopausal osteoporosis. South Med J. 1992;85(Suppl):2-31.).

### Non-porous implant structure

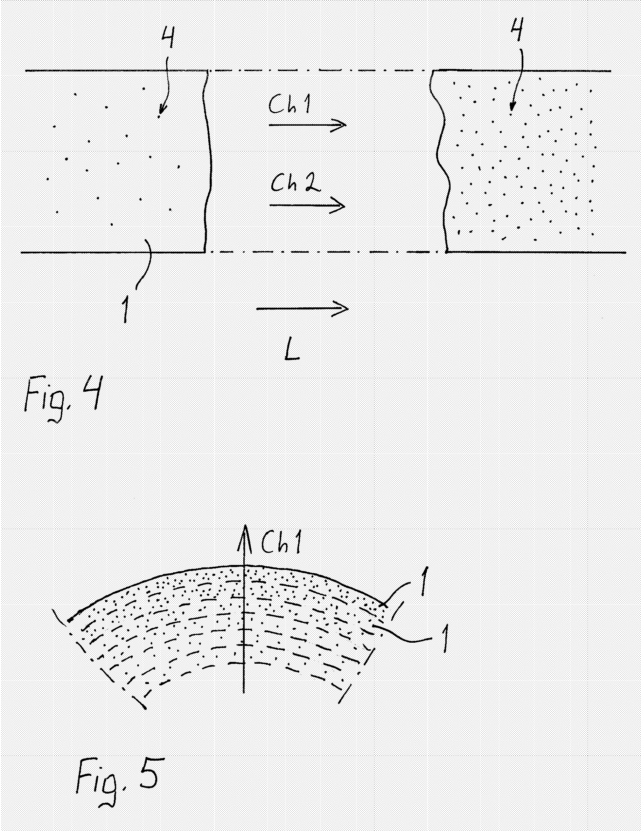

It is also possible to make a substantially non-porous structure from a non-foraminous blank 1 as depicted by Fig. 4. This blank comprises bioabsorbable thermoplastic polymer as matrix and one or several components in the matrix. The component (the other substance) may be for example a bioactive agent or material. It is possible that the bioactive agent is a cytokine and/or pharmaceutical agent or, additionally to or instead of the pharmaceutical agent and/or cytokine, bioactive ceramic such as bioactive glass in fibrous or particulate form. Whatever the type of bioactive agent or material is, its concentration can be used as one characteristic Ch1 varying in the direction of length of the blank L and consequently forming a gradient in the final implant (part of the impant in cross-section in Fig. 5). The concentration of one bioactive material within the matrix is denoted by dots 4 in Fig. 4, where it can be seen that the ratio of the bioactive agent or material to the bioabsorbable polymer (w/w) varies along the length L. It is also possible to use the concentration of another bioactive agent or material as another characteristic Ch2 that varies independenytly of the first one. Finally, the species of the bioactive agent or material can also be used as the characteristic so that a first blank section contains first bioactive agent or material and a second blank section contains second bioactive agent or material. For example two diffferent pharmaceutical agents or two different bioactive ceramics can be placed in the implant so that one of them is closer to the surface and other closer to the core.

The blank 1 can be prepared by melt processing or solution processing method of the matrix polymer, during which the bioactive material can also be incorporated in the matrix so that it will have a desired concentration profile along the length of the blank 1.

The type of bioabsorbable thermoplastic polymer itself can also form a characteristic varying in the length direction of the blank. This can be achieved by feeding different polymer raw material in time-discrete fashion to the blank forming stage. The different polymer raw materials may be of chemically same structure but differing physical properties, for example bioabsorption rate, or they may differ in chemical structure but be compatible with each other so that they can be formed into a cohesive polymer matrix blank. These different polymer may also have differing bioabsorption rate. Thus, the type of matrix polymer forms an additional or only characteristic Ch which can provide a gradient in the final implant, for example in respect of rate of bioabsorption (degradation rate). This characteristic which exists in the blank of Fig. 4 in addition to component concentration characteristics Ch1, Ch2, is denoted Ch3 in Fig. 4.

In the above method, a bioabsorbable implant is achieved which comprises a polymer matrix and a characteristic of the matrix that varies over the cross-section of the implant, by winding a sheet-like blank 1 containing said polymer matrix where said variable varies along a direction L perpendicular to the axis of winding, said cross-section being the result of the winding. Said characteristic is one or several of the following: concentration and/or species of an bioactive agent within said matrix. If the agent is intended to be released into a living body from the implant, its release profile can be accurately adjusted by the concentration gradient of the agent.

If the implant is made by folding according to the principle of Fig. 1, the gradient in the above-mentioned characteristics is created analogically, now from one of the surfaces towards the opposite surface instead of from core to outer surface.

In the following, some details of possible materials for the blank 1 and the final implant are given. These examples are not limiting and can be applied to implant of Fig. 3 or implant of Fig. 5.

### Bioabsorbable thermoplastic polymer

Synthetic bioabsorbable, biocompatible polymers, which may act as suitable materials in the above-mentioned structures can include poly-a-hydroxy acids (e.g. polylactides, polycaprolactones, polyglycolides and their copolymers, such as lactic acid / glycolic acid copolymers and lactic acid / caprolactone copolymers), polyanhydrides, polyorthoesters, polydioxanone, segmented block copolymers of polyethylene glycol and polybutylene terephtalate (Polyactive^{™}), poly(trimethylenecarbonate) copolymers, tyrosine derivative polymers, such as tyrosine-derived polycarbonates, or poly(ester-amides). Suitable bioabsorbable polymers to be used in manufacturing of implants of the present invention are mentioned e.g. in U.S. Pat. Nos. 4,968,317, 5,618,563, FI Patent No. 98136, FI Patent No. 100217B, and in "Biomedical Polymers" edited by S. W. Shalaby, Carl Hanser Verlag, Munich, Vienna, New York, 1994 and in many references cited in the above publications.

The bioabsorbable polymer shall be understood to mean also a blend of two or several different bioabsorbable polymers that differ from each other physically and/or in chemical structure.

### Bioactive ceramic material

A subgroup of bioactive ceramics comprises bioactive glasses. They are surface-active silica-based synthetic biomaterials forming a direct chemical bonding with host tissue i.e., bone. They have the ability to form a calcium phosphate layer on their surface in vivo.

Bioactive glasses have many potential clinical applications. For example, bioactive glass crush can be used as a filler material in bone defects in orthopaedics and in dentistry.

For technically more demanding applications of bioactive glass, i.e., spinning of fibers of bioactive glass, the old generation glasses can not be used due to crystallization of the amorphous material during the spinning procedure which has to be performed at high temperature.

The introduction of a newer generation of bioactive glasses enables the manufacturing of thin bioactive glass fibers. Bioactive glass fibers can be used as a component in a composite consisting of bioabsorbable polymer fibers and bioactive glass fibers. In the composite, bioactive glass functions as a tissue conductive (for example osteoconductive or chondroconductive) material, i.e., for fixation of the implant to host tissue. By changing the oxide composition of the glass, the bioactivity of the material can be controlled enabli ng a tailor-made fixation of the implant to different locations with different tissues and varying physical conditions. Fibrous bioactive glass can be used either as filament or staple fiber.

In another alternative, the bioactive glass can be dispersed in form of particles in the matrix material. In this alternative, bioactive glass without fiber-forming properties can also be used. This type of bioactive glass can be used for example in the substantially non-porous blank 1 of Fig. 4.

Making fibers of bioactive glass is described e.g. in U.S. Patent 6406498 and U.S. Patent 6054400.

The blank 1 may contain calcium phosphates (CPs) in particulate form. The calcium phosphate can be osteoconductive, such as tricalcium phosphate and/or hydroxyapatite. Calcium phosphates (CPs) can be generally classified into two categories, the ones that are obtained by high temperature processes and the ones that can be obtained through basic solution chemistry at ambient temperatures. The high temperature CPs are those that can form CaO-P2O5-H2O system at temperatures beyond 500°C. The examples are monocalcium phosphate alfa- and beta-calcium phosphate, hydroxyapatatite, biphasic calcium phosphate, tetracalcium phosphate and calcium pyrophosphate. A Ca/P ratio varies between 1.0 and 1.67.

Fibers based on CPs are are also included in the present invention. For example, hydroxyapatite can exist also in fibrous form, as taught by US Patent 5,652,056. It can be either as filament or staple fiber, and it can constitute the fibrous bioactive ceramic reinforcing structure. Also tricalcium phosphate fibers can be used, the forming of β-tricalcium phosphate fibers being described e.g. in US Patent 4,655,777.

The CPs described above can constitute the other bioactive ceramic in addition to bioactive glass, and can be incorporated in the other set of fibrous elements running along the length of the blank (such as warp yarns 3b of Fig. 2). The CPs can also be incorporated in the substantially non-porous blank 1 of Fig. 4.

With the manufacturing methods applied in the current invention, it is possible to solve the challenge of having yet a porous structure with interconnected pores or channels that constitute an accurately created gradient related to the macrostructure of the implant. Although these pores may decrease the mechanical properties as compared to solid implant, the bioacive ceramic in fibrous form (staple fibers or filaments) may act as reinforcing elements, and the structure built thereof compensates very much for the strength drop caused by the pores, and the implant produced can be reliable for treatment of tissue defects in load-bearing areas such as those occuring in the bone or cartilage or both of them (osteochondral defects). The device can thus provide mechanical support, structural porosity that can home cells, and osteoconductivity and resorbability over time.

The porous implant may further contain other active agents, such as osteoinductive or antiosteolytic agents, for example embedded in the matrix polymer or in the network of interconnecting pores or channels.

The term "thermoplastic bioabsorbable polymer matrix" includes also blends of two or several thermoplastic bioabsorbable polymers.

The porosity of the structure can be determined by the textile structures, especially how large openings are left between the yarns in the textile structure and what will they sizes be after the final pressing stage. The final structure may have pore size varying at least between 300 to 1000 µm, as measured along the plane of the layers (sections of the blank), the size being sufficient for transport of a wide variety of bioactive agents and cells.

Finally, the porosity of the structure described above can be open, partly open or closed (pores filled partly or completely with material). The pores can be filled completely or partly by a suitable material in the form of powder, non-woven, solution or melt. The material has preferably some bioactive function in the medical device. If the added material is prone to becoming diffused out of the structure through the pores, a bioabsorbable polymer film, which will degrade later in the living body, may be laid over the surface of the porous structure to prevent the diffusion before the implantation. The added material can be synthetic (polymer powder, ceramic powder) or natural (bone graft in powder form, or protein, such as collagen, or polysaccharide or its derivative, such as chitosan). The following section also mentions some materials that can be present in the pores.

### Materials in the pores

Other materials, especially bioactive materials, may be incorporated in the structure in the pores. Pores may containother material than the matrix does. The site of the material depends on the desired function, rate of release and processability (e.g. thermal resistance if the material is to be incorporated in the bioabsorbable matrix polymer by melt processing). Bioactive agents, e.g. cytokines and/or pharmaceutical agents (for example antimicrobial agents), may be impregnated in the porous structure of the medical device in addition to being embedded in the matrix polymer of the fibrous elements of the blank 1.

Furthermore, living cells, e.g., genetically modified or non-modified, can be incorporated in the structure according to need and clinical indication for the treatment of specific problems, especially those of the skeleton occurring in body of mammals following congenital or aquired tissue defects or discontinuities. They can be impregnated or seeded in the porous structure of the implant.

Cytokines, such as appropriate growth factors (e.g., BMPs, angiogenic factors etc.) and/or pharmaceutical agents, agents used for diagnosis or indicators for follow up of treatment and integration, and/or genes/gene vectors, natural and synthetic genetic materials or chromosomes, can be carried in the porous structure of the implant, and consequently bioactive agents are able to exert their effect on the clinical end-applications.

Micro-organisms, e.g., therapeutic bacteria strains, possibly preventing or healing inflammation, can be impregnated into porous structure, which can be implanted in the body. In this case antimicrobial agents are not incorporated in the structure.

### Applications

The structure can have a wide variety of applications especially in osteochondral medical devices. It is designed to be implanted in a living mammalian body. The bioactive and bioabsorbable composite medical device structure can be implanted into human body to induce and/or enhance and/or support hard tissue generation, regeneration, repair or augmentation. Bioactive ceramic component of the implant can be used for fixation of the implant in host tissue. The device of the current invention can be used either alone or in combination with other methods such as fixation with bioabsorbable or metallic screws, tacks, bolts, plates, sutures, etc. Accordingly, the porous, bioactive and bioabsorbable implant structure can be used for tissue repair (e.g., bone fixation), regeneration (e.g., guided bone regeneration) or for tissue generation (e.g., tissue engineering) or augmentation.

After the implant has been implanted in the approproate location in the living body, it has graded functionality by virtue of the gradient towards the outer surface with respect to pore size, bioabsorption (degradation) rate of bioactive ceramic (especially bioactive glass), bioabsorption (degradation) rate of bioabsorbable polymer, concentration of bioactive agent or material (for example pharmaceutical agent or bioactive ceramic), or any combination of these.

It should be noted that the structures shown by the figures may represent the implant body as a whole or they may represent a part of the implant, where other parts of different structure are joined to the structure.

The invention is not solely applicable to hard tissue implants, but the invention can also be applied in the manufacture of soft-tissue implants. For example tubular implants made in accordance with the invention can be used as stents.

## Claims

1. A bioabsorbable implant or its part consisting of a structure having a varying characteristic, in at least in one direction defining a gradient corresponding this characteristic, said structure comprising bioabsorbable polymer and another substance within or next to said bioabsorbable polymer, **characterized in that** the structure is constituted of a wound or folded blank (1) having a direction of variation (L) of a characteristic (Ch) which is at least one of the following: concentration of the other substance, species of the other substance, type of the bioabsorbable polymer, or porosity (size of openings in the blank), said structure having a winding axis or fold lines substantially perpendicular to said direction of variation (L), resulting in said varying characteristic in the structure in said at least one direction defining the gradient and traversing superposed layers formed of sections of the blank (1) that are successive in said direction of variation (L).

2. The bioabsorbable implant or its part according to claim 1, **characterized in that** the structure has at least two different characteristics (Ch) that are arranged independently of each other or are interdependent in said direction defining the gradient.

3. The bioabsorbable implant or its part according to claim 1 or 2, **characterized in that** the other substance is bioactive ceramic.

4. The bioabsorbable implant or its part according to any of the claims 1 to 3, **characterized in that** the characteristic (Ch) is porosity.

5. The bioabsorbable implant or its part according to any of the claims 1 to 4, **characterized in that** the characteristic (Ch) is concentration of the other substance, species of the other substance, or type of the bioabsorbable polymer.

6. The bioabsorbable implant or its part according to claim 5, **characterized in that** one characteristic having the direction of variation is porosity and another characteristic having the direction of variation is the concentration of the other substance, species of the other substance or type of the bioabsorbable polymer.

7. The bioabsorbable implant or its part according to any of claims 3 to 6, **characterized in that** one characteristic having the direction of variation is concentration of bioactive ceramic or species of bioactive ceramic.

8. The bioabsorbable implant or its part according to claim 7, **characterized in that** the bioactive ceramic is bioactive glass.

9. The bioabsorbable implant or its part according to any of the preceding claims, **characterized in that** one characteristic having the direction of variation is type of bioabsorbable polymer.

10. The bioabsorbable implant or its part according to any of the claims 7 to 9, **characterized in that** the characteristic related to bioactive ceramic and/or bioabsorbable polymer results in a gradient in the rate of bioabsorption of bioactive ceramic and/or bioabsorbable polymer.

11. The bioabsorbable implant or its part according to claim 4 or 10, **characterized in that** the porosity increases towards the outer surface of the structure in the direction of the gradient.

12. The bioabsorbable implant or its part according to claim 5, **characterized in that** the other substance is a bioactive agent, such as pharmaceutical agent, and the characteristic having the direction of variation is the concentration or species of the bioactive agent, such as pharmaceutical agent.

13. The bioabsorbable implant or its part according to any of the preceding claims, **characterized in that** the characteristic or a property resulting therefrom is expressable in numerical values and is gradually increasing, gradually decreasing, gradually increasing and then decreasing (showing a maximum), or gradually decreasing and then increasing (showing a minimum) in said direction defining the gradient.

14. The bioabsorbable implant or its part according to any of the preceding claims, **characterized in that** the blank (1) comprises elongate elements (3) defining openings (2) therebetween.

15. The bioabsorbable implant or its part according to claim 14, **characterized in that** the blank (1) contains one set of elongate elements (3a) running in a direction substantially perpendicular to the direction of variation (L) and another set of elongate elements (3b) extending substantially in the direction of variation (L).

16. The bioabsorbable implant or its part according to any of the preceding claims, **characterized in that** the bioabsorbable polymer is thermoplastic polymer and the layers are joined by said thermoplastic polymer.
